(19) **Europäisches Patentamt**
**European Patent Office**
**Office européen des brevets**

(11) Veröffentlichungsnummer: **0 234 404 B1**

## EUROPÄISCHE PATENTSCHRIFT

(45) Veröffentlichungstag der Patentschrift: **29.05.91**

(51) Int. Cl.⁵: **A01K 67/00, A01M 1/00**

(21) Anmeldenummer: **87101833.9**

(22) Anmeldetag: **10.02.87**

(54) **Vorrichtung zur Aufnahme und zum Aufbewahren von Nützlingen.**

(30) Priorität: **18.02.86 CH 649/86**

(43) Veröffentlichungstag der Anmeldung:
**02.09.87 Patentblatt 87/36**

(45) Bekanntmachung des Hinweises auf die
Patenterteilung:
**29.05.91 Patentblatt 91/22**

(84) Benannte Vertragsstaaten:
**AT BE DE FR GB IT LU NL SE**

(56) Entgegenhaltungen:
EP-A- 0 019 168     EP-A- 0 204 999
CH-A- 547 602       DE-A- 2 100 494
DE-A- 3 224 396     DE-C- 209 945
GB-A- 1 196 141     NL-A- 7 707 112
US-A- 3 789 799     US-A- 4 019 709
US-A- 4 368 690

(73) Patentinhaber: **Züricher Beuteltuchfabrik AG**

**CH-8803 Rüschlikon(CH)**

(72) Erfinder: **Müller, Hans Rudolf**
**Ostbühlstr. 49**
**CH-8038 Zürich(CH)**

(74) Vertreter: **EGLI-EUROPEAN PATENT ATTOR-
NEYS**
**Horneggstrasse 4**
**CH-8008 Zürich(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung zur Aufnahme und zum Aufbewahren von Nützlingen nach dem Oberbegriff des Patentanspruches 1.

In der älteren Patentanmeldung EP-A-204999 hat die Anmelderin die Verwendung von Vorrichtungen beansprucht, in denen Nützlinge untergebracht werden können. Diese Vorrichtungen werden dann auf einer Bodenoberfläche ausgelegt, worauf nach einer gewissen Zeit die Nützlinge die Vorrichtung verlassen und die ihnen zugedachte Schädlingsbekämpfung aufnehmen. Die Vorrichtungen können auch an Bäumen oder Sträuchern angebracht werden, wobei sie in diejenige Lage gebracht werden, in welcher sie gegen Witterungseinflüsse, vor allem gegen Regen und Feuchtigkeit geschützt sind.

Die Verteilung der Vorrichtungen erfolgt hierbei in der Weise, dass sie einzeln an die vorgesehene Stelle gebracht werden. Dies ist jedoch nachteilig, wenn es sich darum handelt, grosse Flächen mit solchen Vorrichtungen zu belegen. Damit eine solche Belegung mit vertretbarem Zeitaufwand ausgeführt werden kann, müssen Flugzeuge eingesetzt werden, wie dies bereits aus der Schädlingsbekämpfung mittels chemischen Mitteln bei grossflächigen Räumen bekannt ist.

Aus EP-A-19168 ist eine Vorrichtung zum Anlocken von Nutzinsekten bekannt, welche einen teilweise mit Naturstoffen gefüllten Behälter umfasst, der verschiedene Schlupflöcher aufweist. Ein Schutz gegen Witterungseinflüsse ist jedoch nicht vorgesehen.

Aus DE-C-209945 ist ein Behälter für feuergefährliche Flüssigkeiten bekannt, der bzw. dessen Umhüllung als Stehauf ausgebildet ist. Hierzu muss er oder seine Umhüllung fussseitig beschwert sein. Mit diesem Zusatzgewicht eignet sich der Behälter nicht zum Abwurf aus dem Flugzeug.

Der Erfindung liegt die Aufgabe zugrunde, die eingangs beschriebene Vorrichtung so auszugestalten, dass sie bei grossflächigem Einsatz in grosser Zahl wie bei der chemischen Schädlingsbekämpfung aus dem Flugzeug abgeworfen werden kann, wobei jedoch die Vorrichtungen auch gegen Witterungseinflüsse geschützt sind.

Diese Aufgabe wird gemäss der Erfindung durch das Kennzeichen des Patentanspruches 1 gelöst.

Die Erfindung ist in der Zeichnung in Ausführungsbeispielen dargestellt und nachfolgend beschrieben. Es zeigen:

Fig. 1    eine Vorrichtung zur Aufnahme von Nützlingen, bei welcher das Gehäuse der Vorrichtung zur Aufnahme der Nützlinge mit Stützstäben versehen ist, welche eine Berührung des Gehäuses mit dem Boden verhindern,

Fig. 2    eine ähnliche Vorrichtung wie in Fig. 1, jedoch mit einem zylinderischen Gehäuse,

Fig. 3    eine dreiteilige Vorrichtung in auseinandergezogener Darstellung, wobei die stirnseitigen Abschlüsse des zylinderförmigen Gehäuses als Scheiben ausgebildet sind,

Fig. 4    eine weitere Ausführungsform einer Scheibe für eine Vorrichtung nach Fig. 3,

Fig. 5    eine Vorrichtung mit einem kugelförmigen Gehäuse und daran befestigten Stützelementen,

Fig. 6    eine weitere Ausführungsform eines kugelförmigen Gehäuses,

Fig. 7    ein schematisch dargestellter Schnitt des Gehäuses einer Vorrichtung, in welchem Nützlinge eingelgt sind,

Fig. 8    eine schematische Darstellung eines Trägers für die Lagerung von Nützlingen zum Einlegen in ein Gehäuse nach Fig. 7 und

Fig. 9    eine Kette von zusammenhängenden, mit Abstand angeordneten Vorrichtungen.

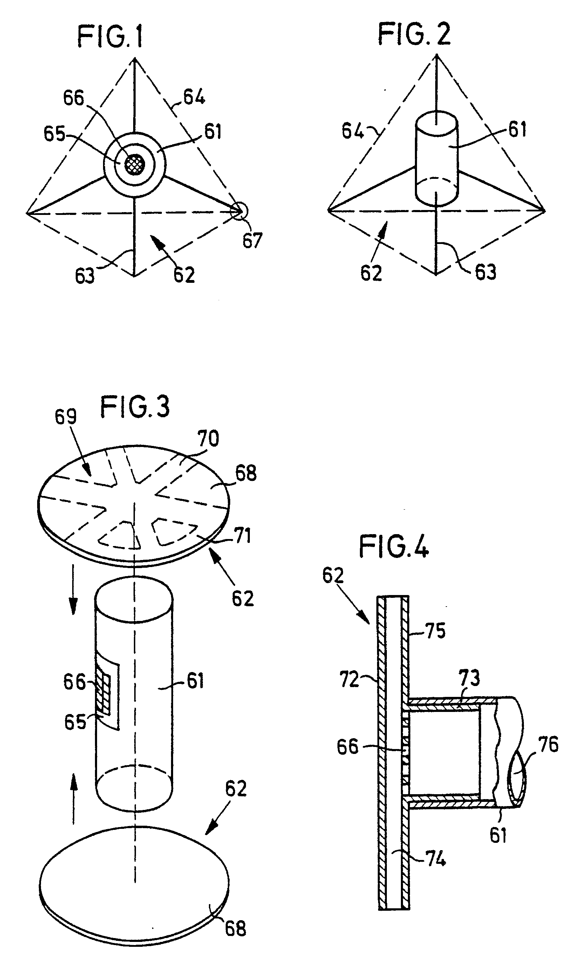

Die in Fig. 1 dargestellte Vorrichtung weist ein Gehäuse 61 auf, an deren Aussenfläche 3 abragende Stützglieder 62 befestigt sind. Die Stützglieder 62 sind beispielsweise Stäbe 63, welche vom Zentrum gegen die Ecken eines gedachten, gestrichelt angedeuteten Tetraeders 64 gerichtet sind. Das Gehäuse 61 der Vorrichtung weist einen Wandteil 65 auf, in welchem eine Anzahl Oeffnungen 66 mit definierter Querschnittfläche vorgesehen sind. Bezüglich der Details der Ausbildung des Gehäuses 61 und des Wandteils 65 wird auf die eingangs erwähnte ältere Patentanmeldung verwiesen. Die in Fig. 1 als Stützglieder 62 dargestellten Stäbe können auch als schmale Stege oder als Stäbe mit einer Endplatte 67, siehe Fig. 1, ausgebildet sein. Der Zweck der Stützglieder 62, wie sie nachstehend in verschiedenen Ausführungsformen dargestellt werden, dienen dazu, das Gehäuse 61 unabhängig von der jeweiligen Lage der Vorrichtung so zu halten, dass das Gehäuse 61 nicht mit dem Boden in Berührung kommt. Ein Bodenkontakt wäre für die im Gehäuse 61 untergebrachten Nützlinge schädlich, wenn dadurch Feuchtigkeit in das Gehäuseinnere eintreten könnte. Da dies durch die Stützglieder 62 vermieden wird, kann die Vorrichtung nach Fig. 1 auf grossen Flächen mittels Abwurf aus Flugzeugen verteilt werden. Durch die Stützglieder 62 wird zuverlässig ein Bodenkontakt des Gehäuses 61 und damit ein Schaden an den Nützlingen vermieden.

Während in Fig. 1 das Gehäuse 61 Hohlkugelform aufweist, ist das Gehäuse 61 der Vorrichtung nach Fig. 2 als Hohlzylinder ausgebildet. Die Stützglieder 62, bei dieser Vorrichtung als Stäbe 63 ausgebildet, sind, wie in Fig. 1, vom Zentrum gegen die Ecken eines gedachten Tetraeders 64 gerichtet. Das Gehäuse 61 weist ebenfalls einen Wandteil mit definierten Oeffnungen (nicht dargestellt) auf und ebenso können auch anstelle der Stäbe 63 andere Stützglieder 62 verwendet werden, welche Gewähr bieten, dass beim Abwurf der Vorrichtung aus dem Flugzeug das Gehäuse 61 in jeder Lage keinen Bodenkontakt aufweist. Es können auch mehr als vier Stützglieder 62 vorgesehen werden, jedoch stellt die in Fig. 1 und 2 dargestellte Verwendung von drei Stützgliedern 62 eine bezüglich ihres Aufwandes optimale Lösung dar. Bei der Verwendung von Stäben 63 ist auch ein Zusammenhaken der Vorrichtungen nicht zu erwarten.

Aus Fig. 3 ist ersichtlich, dass die Vorrichtung sich aus drei Teilen zusammensetzt, aus denen die Vorrichtung gebildet wird: einem Gehäuse 61 in Hohlzylinderform und zwei Stützgliedern 62, welche als Folien 68 in Kreisflächenform ausgebildet sind. Die Pfeile bezeichnen den Zusammenbau der Vorrichtung, wobei die Scheiben 68 die Stirnseiten des hohlzylinderförmigen Gehäuses abdecken. In der Mantelfläche des Gehäuses 61 ist ein Wandteil 65 mit den Oeffnungen 66 angedeutet. Auch die Vorrichtung nach Fig. 3 eignet sich dazu, in grösseren Mengen aus dem Flugzeug abgeworfen und auf grossen Flächen verteilt zu werden. Auch in diesem Fall kann mit grosser Wahrscheinlichkeit damit gerechnet werden, dass die im Gehäuse eingebrachten Nützlinge durch Feuchtigkeit nicht beschädigt werden. Anstelle der Scheiben 68 können die Stützglieder 62 auch anders geformt sein. In der einen Scheibe 68 in Fig. 3 ist gestrichelt eine Speichenscheibe als Variante dargestellt, deren Speichen 70 entweder frei enden oder durch einen Speichenring 71 zusammengehalten sind.

In Fig. 4 ist eine Vorrichtung teilweise dargestellt, welche als Stützglied 62 zwei Scheiben 72 (nur eine gezeichnet) aufweist. Die Scheibe 72 weist einen Rohrstutzen 73 im Zentrum der Scheibe 72 auf, welcher zur Montage der Vorrichtung in das Ende des hohlzylinderförmigen Gehäuses 61 gesteckt wird. Die Scheibe 72 weist einen spaltförmigen Kanal 74 auf, welcher sich diagonal über die Scheibe 72 erstreckt. Die den Rohrstutzen 73 tragende Seitenfläche 75 weist im Bereich des Rohrstutzens 73 Oeffnungen 66 auf, durch welche Nützlinge zu gegebener Zeit das Gehäuse 61 verlassen. Der Rohrstutzen 73 dient dazu, die Nützlinge in einem gewissen Abstand von dem Stützglied 62 zu halten. Fällt die Vorrichtung beim Abwurf aus dem Flugzeug so, dass sie auf einem Stützglied 62, d.h. auf einer Scheibe 72, stehen bleibt, werden auch in diesem Fall die Nützlinge mit Abstand von durch den Kanal 74 eindringende Feuchtigkeit im Hohlraum des Gehäuses 61 zurückgehalten.

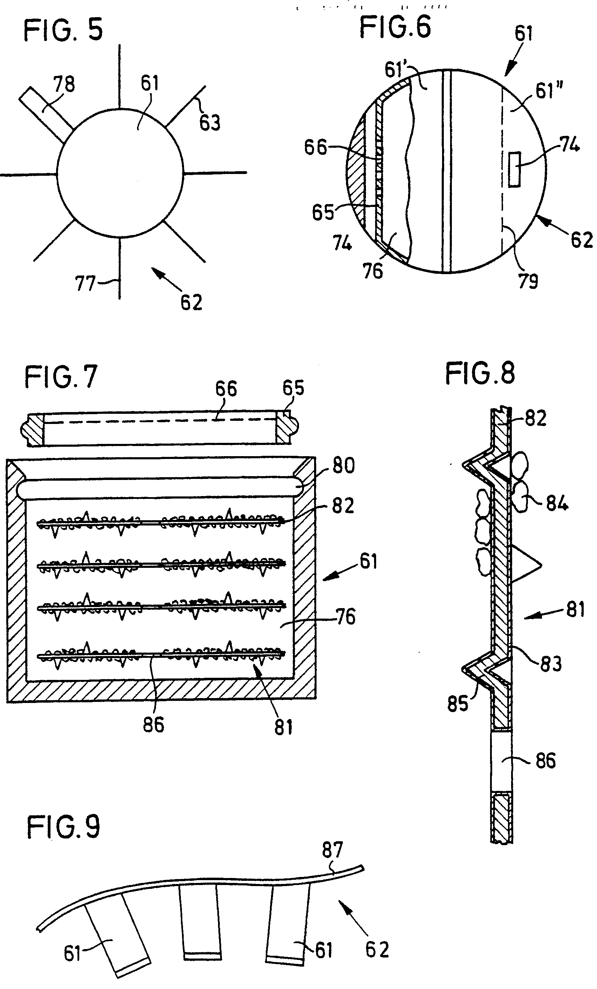

In Fig. 5 ist eine Vorrichtung mit einem kugelförmigen Gehäuse 61 dargestellt, auf dessen Oberfläche radial abragende Stützglieder 62 befestigt sind. Die Stützglieder 62 können verschiedene Formen aufweisen und als Stäbe 63, ringförmige Scheiben 77 oder Stege 78 ausgebildet sein. Gewöhnlich wird jedoch nur eine dieser Ausführungsformen bei einer Vorrichtung nach Fig. 5 angewandt.

In Fig. 6 ist ein hohlkugelförmiges Gehäuse 61, teilweise im Schnitt, dargestellt. Das Gehäuse 61 setzt sich aus zwei Halbkugelschalen 61', 61'' zusammen. Aus dem Schnitt auf dem linken Teil von Fig. 6 ist erkennbar, dass ein spaltförmiger Kanal 74 vorgesehen ist, an welchem ein Wandteil 65 mit Oeffnungen 66 anliegt, wobei die Oeffnungen 66 die Verbindung zwischen dem Hohlraum 76, in welchem die Nützlinge untergebracht sind, und dem spaltförmigen Kanal 74 bilden. Die beiden Gehäusehälften 61', 61'' werden nach dem Einbringen der Nützlinge zweckmässig so zusammengefügt, dass die Kanäle 74 um 90 Grad zueinander liegen. Werden nun Vorrichtungen nach Fig. 6 mittels Abwurf von dem Flugzeug auf Flächen verteilt, wird auch durch diese Ausführungsform erreicht, dass die Nützlinge in beliebiger Lage der Vorrichtung nicht durch Feuchtigkeit ungünstig beeinflusst oder sogar beschädigt werden.

Bei der Ausführung nach Fig. 6 sind zwar keine von der Aussenfläche des Gehäuses 61 abragenden Teile vorgesehen, jedoch bildet das im rechten Teil von Fig. 6 durch eine gestrichelte Linie 79 abgetrennte Kugelsegment das Stützglied 62, obwohl es mit dem betreffenden Gehäuseteil 61', 61'' einen einzigen Teil bildet. Allerdings wäre es auch möglich, das Gehäuse 61 einteilig und die Stützglieder 62 davon getrennt auszubilden. Es würde dann eine Ausführung vorliegen, wie sie etwa in Fig. 4 dargestellt ist.

Fig. 7 zeigt ein hohlzylinderförmiges Gehäuse 61 mit einem, Oeffnungen 66 aufweisenden Wandteil 65. Nachdem die Nützlinge im Hohlraum des Gehäuses 61 untergebracht sind, kann der Wandteil 65 in eine Nut 80 eingeschnappt werden, welche an der Innenwand des Gehäuses 61 angeordnet ist. Am Gehäuse 61 sind Stützglieder 62 in der beschriebenen Weise angeordnet, die jedoch der Einfachheit halber hier nicht dargestellt sind.

Die Unterbringung der Nützlinge ist aus Fig. 7 und 8 ersichtlich. Es werden hierzu Träger 81, z.B. in Form von Scheiben 82 verwendet. Diese werden mit einem Haftmittel, z.B. einem Klebstoff, beschichtet, worauf dann die Nützlinge 84, z.B. Eier, Larven und dgl. auf die Scheibe aufgebracht werden. Damit zwischen den einzelnen Scheiben 82 genügend Platz für die Nützlinge 84 vorhanden ist, werden an den Scheiben einseitig oder beidseitig Distanzhalteelemente 85 angeordnet. Wie aus Fig. 8 ersichtlich ist, sind die Distanzhalteelemente 85 in die Scheiben 82 eingeprägt, so dass die Distanzhalteelemente 85 kegelförmige Form aufweisen. In der Mitte jeder Scheibe 82 ist ein Durchgang 86 vorgesehen, damit die Nützlinge 84 zu den Oeffnungen 66 gelangen können. Die Träger 81 für die Nützlinge 84 werden, siehe Fig. 7, in den Hohlraum 76 des Gehäuses 61 geschichtet, worauf der Hohlraum 76 mit dem Wandteil 65 gesperrt wird. Damit ist die aus dem Gehäuse 61 und den Stützgliedern 62 bestehende Vorrichtung für den Abwurf aus dem Flugzeug bereit. In analoger Weise werden die Vorrichtungen gemäss Fig. 1-6 vorbereitet.

In Fig. 9 ist eine weitere Form eines Stützgliedes 62 dargestellt, welches die Form eines Fadens 87 aufweist, an dem mit Abstand die Gehäuse 61 befestigt sind. Da der Faden 87 und die Gehäuse 61 aus Kunststoff bestehen, kann mit der Herstellung der Gehäuse 61 gleichzeitig die Verbindung mit dem Faden oder der Schnur 87 vorgenommen werden, so dass die Herstellung solcher Gehäuseketten praktisch ohne zusätzlichen Aufwand möglich ist. Solche Gehäuseketten werden vorzugsweise durch Abwurf aus dem Flugzeug auf Gebieten verteilt, auf denen Gebüsch oder Bäume wachsen. Die Gehäuseketten verfangen sich dabei im Geäst, wodurch der Schutz der Nützlinge gegen Feuchtigkeit erreicht wird.

Durch die beschriebenen Vorrichtungen wird erreicht, dass der Einsatz von Nützlingen auch auf sehr grossen Gebieten möglich ist. Durch die besondere Ausbildung der Vorrichtungen wird erreicht, dass die Nützlinge auch beim Abwurf aus dem Flugzeug unbeschädigt zum Einsatz kommen können. Als Material für die Vorrichtungen ist ein Kunststoff zu verwenden, der keine umweltbelastenden Komponenten aufweist und in einer gewissen Zeit gegebenenfalls durch eine entsprechende Behandlung abbaut. Es sei hierzu auf die eingangs erwähnte Patentanmeldung verwiesen.

**Ansprüche**

1. Vorrichtung zur Aufnahme und zum Aufbewahren von Nützlingen für die Beeinflussung der Umwelt, durch Bekämpfung und Vernichtung von schadenerzeugenden bakteriellen und anderen Organismen sowie Materialien, wobei die Nützlinge in einem als Hohlraum (76) ausgebildeten Gehäuse (61) mit einer der Körpergrösse der Nützlinge angepassten Öffnung (66) untergebracht sind, dadurch gekennzeichnet, dass das als Hohlraum (76) ausgebildete Gehäuse (61) Stützmittel (62) aufweist, deren Form den die Nützlinge (84) aufnehmenden Hohlraum (76) derart abdeckt oder überragt, dass der Hohlraum unabhängig von der jeweiligen Lage des Gehäuses von der Unterlage, auf welcher sich die Stützmittel abstützen, mit Abstand gehalten ist.

2. Vorrichtung nach Anspruch 1, dadurch gekennzeichnet, dass die Stützmittel (62) Stützglieder, z.B. Stäbe (63) oder Stege, umfassen, welche von der Oberfläche des kugel- oder zylinderförmigen Gehäuses (61) abragen.

3. Vorrichtung nach Anspruch 1, dadurch gekennzeichnet, dass die Stützmittel (62) das Gehäuse (61) überragende Stützscheiben (68) sind, welche vorzugsweise in gegenüberliegender Lage angeordnet sind.

4. Vorrichtung nach Anspruch 3, dadurch gekennzeichnet, dass in den Stützscheiben (72) ein längs zur Scheibenoberfläche verlaufender Kanal (74) vorgesehen ist, in dessen Innern eine Verbindung zur Öffnung (66) des Gehäuses (61) vorgesehen ist.

5. Vorrichtung nach Anspruch 1, dadurch gekennzeichnet, dass als Stützmittel ein an der Wand des Gehäuses (61) angeordnetes, mit dem Kanal (74) versehenes Stützglied (62) vorgesehen ist, wobei die Öffnung (66) des Hohlraumes (74) des Gehäuses im Innern des Kanals mündet.

6. Vorrichtung nach Anspruch 1, dadurch gekennzeichnet, dass in dem Hohlraum (76) des Gehäuses (61) Trägermittel (81) zur Lagerung der Nützlinge (84) in Form übereinander geschichteter, scheibenförmiger Folien (82) eingelegt sind, welche mit einem Durchgang (86) versehen sind und auf deren Seitenflächen eine Anzahl Distanzhalteelemente (85) zur Einhaltung eines Mindestabstandes zwischen den Folien angeordnet sind.

7. Vorrichtung nach Anspruch 6, dadurch gekennzeichnet, dass die Folien (82) mindestens auf einer der beiden Seitenflächen mit Haftmitteln (83), z.B. einem Klebstoff belegt sind, mit Hilfe derer die Nützlinge (84) auf der Folienoberfläche mindestens zeitweise festgehalten sind.

8. Vorrichtung nach Anspruch 1, dadurch gekennzeichnet, dass an dem Gehäuse (61) ein Faden (87) befestigt ist, an welchem weitere Gehäuse befestigt sind, zwecks Bildung von Gehäuseketten.

## Claims

1. Device for receiving and storing useful organisms for influencing the environment by combatting and removal of damage generating bacteria and other organisms as well as materials, wherein the useful organisms are introduced into a housing (61) constructed as a hollow space (76) with an opening (66) matched to the body size of the useful organisms, characterised in that the housing (61) constructed as a hollow space (76) has support means (62), the shape of which so covers or projects from the shape of the hollow space (76) receiving the useful organisms (84) in such a fashion that the hollow space is held at a distance from the substrate on which the supporting elements rest irrespective of the particular position of the housing.

2. Device according to Claim 1, characterised in that the support means (62) comprise support member, e.g. bars (63) or tabs which project from the surface of the spherical or cylindrically shaped housing (61).

3. Device according to Claim 1, characterised in that the support means (62) are support discs (68) projecting from the housing (61) which are preferably arranged in opposed positions.

4. Device according to Claim 3, characterised in that in the support discs (72); there is provided a channel (74) running longitudinally relative to the disc surface in the interior of which is provided a connection to the opening (66) of the housing (61).

5. Device according to Claim 1, characterised in that, as support means, there is provided a support member (62) provided with the channel (74) arranged on the wall of the housing (61), wherein the opening (66) debouches into the hollow space (74) of the housing in the interior of the channel.

6. Device according to Claim 1, characterised in that the carrier means (81) in the hollow space (76) of the housing (61) for storing the useful organisms (84) are introduced in the form of disc shaped sheets (82) layered over one another which are provided with a passage (86) and on the side surfaces of which a number of distance maintaining elements (85) are arranged for maintaining a minimum distance between the sheets.

7. Device according to Claim 6, characterised in that the sheets (82) are coated at least on one of both side surfaces with adhesive agents (83), e.g. of glue, with the aid of which the useful organisms (84) are fixed at least temporarily to the sheet surface.

8. Device according to Claim 1, characterised in that a thread (87) is fixed to the housing (61) to which further housings are fixed with the object of forming housing chains.

## Revendications

1. Dispositif destiné à loger et à garder des organismes utiles pour influencer l'environnement par la lutte et la destruction d'organismes bactériens et autres organismes ainsi que des matières nuisibles, les organismes utiles étant enfermés dans une boîte (61) en forme de cavité (76) présentant une ouverture (66), adaptée à la taille des organismes utiles, caractérisé en ce que la boîte (61), configurée en cavité (76), comporte des moyens d'appui (62) dont la forme recouvre ou dépasse de la cavité (76) logeant les organismes utiles (84), en ce que la cavité est maintenue à une distance du support sur lequel prennent appui les moyens d'appui, indépendamment de la position de la boîte.

2. Dispositif selon la revendication 1, caractérisé en ce que les moyens d'appui (62) comportent des organes d'appui, par exemple des barres (63) ou des étais qui dépassent de la surface de la boîte (61) sphérique ou cylindrique.

3. Dispositif selon la revendication 1, caractérisé en ce que les moyens d'appui (62) sont des disques d'appui (68) dépassant de la boîte (61) qui, de préférence, se font face.

4. Dispositif selon la revendication 3, caractérisé en ce que dans les disques d'appui (72), il est prévu un canal (74) s'étendant le long de la surface des disques, à l'intérieur duquel il est prévu une communication avec l'ouverture (66) de la boîte (61).

5. Dispositif selon la revendication 1, caractérisé en ce qu'il est prévu comme moyen d'appui, un organe d'appui (62) placé contre la paroi de la boîte (61), pourvu du canal (74), l'ouverture (66) de la cavité (74) de la boîte débouchant à l'intérieur du canal.

6. Dispositif selon la revendication 1, caractérisé en ce que dans la cavité (76) de la boîte (61), sont placés des moyens de support (81), destinés au stockage des organismes utiles (84) sous la forme de feuilles (82) en forme de disque, superposés qui sont pourvues d'un passage (86) et sur les faces latérales desquelles est disposé un certain nombre d'éléments d'écartement (85), destinés à faire respecter une distance minimale entre les feuilles.

7. Dispositif selon la revendication 6, caractérisé en ce que les feuilles (82) sont recouvertes au moins sur l'une des deux faces latérales de moyens d'adhérence (83), par exemple d'une colle, à l'aide desquels les organismes utiles (84) sont maintenus, au moins provisoirement, sur la surface des feuilles.

8. Dispositif selon la revendication 1, caractérisé en ce qu'un fil (87), auquel sont attachées d'autres boîtes, est fixé sur la boîte (61), pour former des chaînes de boîtes.

FIG.1

FIG.2

FIG.3

FIG.4

7

FIG. 5

FIG.6

FIG.7

FIG.8

FIG.9